# EUROPÄISCHE PATENTSCHRIFT

(11) **EP 3 589 154 B1**
(45) Veröffentlichungstag und Bekanntmachung des Hinweises auf die Patenterteilung: **27.04.2022**
(21) Anmeldenummer: 18707920.7
(22) Anmeldetag: 02.03.2018
(51) Int. Cl.: A44C 17/00

(54) **VERFAHREN ZUM RÜCKVERFOLGBARMACHEN EINES SCHMUCKSTEINS**
METHOD FOR MAKING A GEMSTONE TRACEABLE
PROCÉDÉ PERMETTANT LA TRAÇABILITÉ D'UNE PIERRE PRÉCIEUSE

(30) Priorität: 02.03.2017 CH 2582017
(43) Veröffentlichungstag der Anmeldung: 08.01.2020
(73) Patentinhaber: Gübelin Gem Lab Ltd., 6006 Luzern (CH)
(72) Erfinder: NYFELER, Daniel, 4933 Rütschelen (CH); LINK, Klemens, 6102 Malters (CH)
(74) Vertreter: Frei Patent Attorneys
(86) Internationale Anmeldenummer: PCT/EP2018/055212
(87) Internationale Veröffentlichungsnummer: WO 2018/158444

(56) Entgegenhaltungen:
- WO-A1-2006/053435
- WO-A2-02/086052
- GB-A- 2 387 437
- US-A1- 2005 019 556
- US-A1- 2009 286 250
- US-A1- 2015 107 475

## Beschreibung

Die Erfindung bezieht sich auf das Gebiet von Verfahren zum Rückverfolgbarmachen von Schmucksteinen. Das Rückverfolgbarmachen ermöglicht eine Rückverfolgung eines Schmucksteins. Die Rückverfolgung von Schmucksteinen ist für Abnehmer von Schmucksteinen eine wichtige Information.

Durch die Rückverfolgung kann beispielsweise ermittelt werden, woher ein Schmuckstein stammt. Auf diese Weise sind Rückschlüsse auf spezifische Minen und/oder geographische Gebiete möglich. Auch Rückschlüsse auf eine Herkunft aus offiziellen Schürf- und/oder Vertriebskanälen ist möglich. Eine Bearbeitung des Schmucksteins kann beispielsweise ebenfalls von der Rückverfolgung nachvollziehbar sein. Je nach Anwendung eines Verfahrens zur Rückverfolgung kann also durch die Rückverfolgung in Erfahrung gebracht werden, woher ein Schmuckstein stammt, durch welche Hände er gegangen ist und/oder wann und wo er bearbeitet worden ist.

Durch die Rückverfolgung kann verhindert werden, dass gestohlene, gefälschte und/oder geschmuggelte Schmucksteine wie normale Schmucksteine gehandelt werden. Auch Schmucksteine aus illegalen Minen, aus Minen mit ausbeuterischen Rahmenbedingungen und/oder aus Minen in Kriegsgebieten können erkannt werden. Die Rückverfolgung von Schmucksteinen kann somit negative Aspekte des Geschäfts mit Schmucksteinen mindern oder beseitigen. Die Rückverfolgung kann aber auch den Wert von Schmucksteinen durch die Belegbarkeit von deren Herkunft erhöhen. Die Rückverfolgung von Schmucksteinen gewinnt im globalisierten Handel zunehmend an Bedeutung. Deswegen ist ein Rückverfolgbarmachen eines Schmucksteins für viele verschiedene Parteien von Interesse.

Verschiedene derartige Verfahren zur Rückverfolgung von Schmucksteinen sind beispielsweise aus WO0210091A2 bekannt. Der darin beschriebene Stand der Technik von Verfahren zum Rückverfolgbarmachen umfasst (Laser-)Gravur und Ätzmethoden zum Markieren von Schmucksteinen. Aber dies beeinträchtigt und/oder beschädigt den Schmuckstein. Das in WO0210091A2 beschriebene Verfahren beinhaltet eine entfernbare Umhüllung der Schmucksteine, insbesondere mit einer Polymerhülle. Die Hülle kann dann eindeutig identifizierbare Eigenschaften aufweisen, etwa durch eine bestimmte chemische Zusammensetzung der Hülle und/oder durch Markierungen der Hülle auf verschiedener Art und Weise (beispielsweise Strichcodes, Hologramme, Matrizen, fluoreszierende Farbteilchen etc.). GB2387437A beschreibt eine Methode zur Rückverfolgung von Gegenständen mittels verschiedener Kombinationen von Oligomeren. WO2006053435A1 beschreibt eine Methode zur Rückverfolgung von Gegenständen mittels Markern mit einzigartigen optischen Eigenschaften und/oder umfassend DNA. US2005019556A1 beschreibt eine Methode zum Markieren eines metallischen Objekts, dessen Markierung mit einem optischen Mikroskop sichtbar ist.

Der Nachteil der bekannten Methoden ist, dass ein Schmuckstein beim Rückverfolgbarmachen beeinträchtigt oder sogar beschädigt wird. Oder der Schmuckstein wird mit einer Methode rückverfolgbar gemacht, welche ein Entfernen der Identifizierungsmöglichkeit (beispielsweise der Polymerhülle) erlaubt. Durch ein Entfernen der Identifizierungsmöglichkeit ist eine Manipulation der Rückverfolgung möglich. Eine derartige Methode ist nicht sehr sicher und kann unzuverlässig sein.

Ein anderer Nachteil der bekannten Methoden ist, dass die Schmucksteine dadurch in ihrer optischen Qualität beeinträchtigt sein können. Anders gesagt können bekannte Methoden des Rückverfolgbarmachens an einem Schmuckstein von menschlichem Auge sichtbare Veränderungen verursachen. Beispielsweise werden Strichcodes oder Umhülllungen am Schmuckstein angebracht. Von blossem Auge sichtbare Veränderungen mindern aber den Wert eines Schmucksteins, besonders wenn dieser bereits fertig verarbeitet ist.

Ein weiterer Nachteil der bekannten Methoden zum Rückverfolgbarmachen ist, dass die Rückverfolgbarkeit eines Schmucksteins nach einer Verarbeitung des Schmucksteins beeinträchtigt oder sogar unmöglich ist. Beispielsweise kann nach einem Schleifen oder Schneiden des Schmucksteins eine gravierte oder geätzte Markierung teilweise oder ganz beschädigt oder sogar entfernt sein. Eine Hülle des Schmucksteins kann beispielsweise bei einem Brennen, Bestrahlen, Schleifen, Schneiden, Ölen, Fetten, Paraffinieren und/oder Wachsen ebenfalls teilweise oder ganz beschädigt oder sogar entfernt werden. Ein Schmuckstein kann beispielsweise mit bekannten Methoden je nach Methode nur schlecht oder sogar gar nicht in einem fertig verarbeiteten Zustand (mit oder ohne Fassung) rückverfolgt werden.

Es ist deshalb Aufgabe der Erfindung, ein Verfahren zum Rückverfolgbarmachen von Schmucksteinen zu schaffen, welche mindesten einen der oben genannten Nachteile mindestens teilweise behebt.

Diese Aufgabe löst ein Verfahren mit den Merkmalen des Patentanspruches 1.

Das erfindungsgemässe Verfahren zum Rückverfolgbarmachen eines Schmucksteins umfasst die Schritte:
zur Verfügung stellen eines unsichtbaren Markers, welcher eine Grösse von 10 nm bis und mit 1000 nm aufweist, wobei der Marker eindeutig identifizierbar ist,
Transport des in einer Trägerflüssigkeit suspendierten Markers zu einer Einlagerungsstelle, um den Marker in der Einlagerungsstelle des Schmucksteins zu befestigen,
wobei der Schmuckstein mindestens teilweise in die Trägerflüssigkeit mit dem suspendierten Marker eingetaucht wird, um den in der Trägerflüssigkeit suspendierten Marker zur Einlagerungsstelle im Schmuckstein zu transportieren, wobei die Trägerflüssigkeit sich in einem Gefäss befindet,
wobei der Schmuckstein über eine Zeitdauer von mehreren Stunden mindestens teilweise in der Trägerflüssigkeit eingetaucht ist und zusätzlich der Schmuckstein und die Trägerflüssigkeit in Bewegung versetzt werden, also beispielsweise geschüttelt, beschallt (insbesondere mit Ultraschall), geschwenkt, rotiert oder gerührt,
Befestigen des Markers in der Einlagerungsstelle des Schmucksteins, und
Erfassen einer Markierungsinformation, welche beinhaltet, dass dieser eindeutig identifizierbare Marker an diesem Schmuckstein befestigt ist.

Ein Schmuckstein wird manchmal auch als Edelstein bezeichnet. Ein Schmuckstein ist beispielsweise ein Smaragd, ein Diamant, ein Rubin, ein Saphir, ein Korund, ein Achat, ein Beryll, ein Malachit, ein Turmalin oder ein Topas. Unter die Bezeichnung Schmuckstein fällt ein Edelstein ebenso wie ein Halbedelstein. Ein Schmuckstein kann ein Mineral, ein Gestein, eine Glasschmelze oder ein Stoff organischer Herkunft sein (wie beispielsweise Bernstein, Pechkohle oder ein Fossil bzw. Fossilstück). Auch Perlen, Perlmutt und Korallen zählen zu den Schmucksteinen.

Der Schmuckstein ist insbesondere natürlichen Ursprungs.

Mit natürlichem Ursprung ist gemeint, dass der Schmuckstein nicht künstlich hergestellt ist. Mit anderen Worten ist ein Schmuckstein natürlichen Ursprungs in der freien Natur entstanden und auffindbar. Beispielsweise wird ein Schmuckstein natürlichen Ursprungs in Minen geschürft. Alternativ ist der Schmuckstein synthetisch hergestellt.

Insbesondere ist der Schmuckstein ein Beryll und insbesondere ein Smaragd, also ein grüner Beryll.

Ein Marker ist ein sich räumlich in drei Dimensionen erstreckendes physisches Objekt. Ein unsichtbarer Marker ist mit blossem menschlichem Auge nicht sichtbar. Mit anderen Worten wird der Marker als unsichtbar bezeichnet, wenn ein Mensch den Marker ohne Hilfsmittel nicht sehen kann. Der Marker kann beispielsweise aufgrund von Grösse, Brechungsindex, Dichte, Transparenz und/oder Reflektion für das blosse menschliche Auge unsichtbar sein.

Insbesondere ist der Marker auch mit optischen Instrumenten nicht sichtbar.

Mit optischen Instrumenten sind Instrumente gemeint, welche eine Bildvergrösserung ausschliesslich auf optischem Weg erzielen, also durch Verwendung von optischen Linsen. Optische Instrumente sind beispielsweise

Brillen, Lupen oder Lichtmikroskope, also lichtoptische Geräte und Instrumente. Hingegen sind Elektronenmikroskope oder Rastersondenmikroskope explizit keine optischen Instrumente im Rahmen dieser Anmeldung.

Ein Marker weist eine charakteristische Eigenschaft auf, welche eine eindeutige Identifizierung des Markers erlaubt. Eindeutig identifizierbar ist der Marker, wenn klar festgestellt werden kann, dass es sich um genau einen spezifischen Marker handelt. Die charakteristische Eigenschaft des Markers kann eine einzelne Eigenschaft sein oder aus einer Kombination von spezifischen Eigenschaften bestehen.

Beispielsweise kann ein Marker DNS umfassen, radioaktiv strahlende Bestandteile umfassen, Isotopen umfassen, Spurenelemente umfassen, Metalle der seltenen Erden umfassen, polarisiert sein, elektrisch geladen sein, magnetisiert sein, einen bestimmten Quantenzustand aufweisen, fluoreszierende und/oder phosphoreszierende Bestandteile umfassen und/oder eine spezifische chemische Signatur aufweisen und aufgrund einer oder mehrerer dieser Eigenschaften eindeutig identifizierbar sein.

Insbesondere kann die charakteristische Eigenschaft beinhalten, dass der Marker ein Isotop umfasst.

Insbesondere kann die charakteristische Eigenschaft beinhalten, dass der Marker mehrere Isotope in einem spezifischen Mengenverhältnis zueinander umfasst.

Insbesondere kann die charakteristische Eigenschaft beinhalten, dass der Marker ein Metall der seltenen Erden (REE) umfasst.

Die charakteristische Eigenschaft des Markers kann beispielsweise mindestens 50 unterschiedliche und voneinander klar unterscheidbare Werte oder Identitäten aufweisen. Beispielsweise kann die charakteristische Eigenschaft des Markers mindestens 100 unterschiedliche Werte aufweisen. Insbesondere kann die charakteristische Eigenschaft des Markers mindestens 500 unterschiedliche Werte aufweisen. Damit ist gemeint, dass durch den Marker eine Identifizierung und Unterscheidung von mindestens 50 bzw. 100 oder 500 Markern derselben Art (d.h. mit derselben charakteristischen Eigenschaft) möglich ist. Eine positive oder eine negative elektrische Ladung alleine reicht also nicht, um einen Marker im Sinne des in diesem Absatz beschriebenen Beispiels eindeutig zu identifizieren.

Ein Marker erlaubt beispielsweise ein Kennzeichnen und Identifizieren eines Objekts, welchem der Marker zugeordnet ist. Ein Objekt, dem ein Marker zugeordnet ist, kann auf diese Weise erkannt und rückverfolgt werden. Durch einen Marker kann ein Schmuckstein rückverfolgbar gemacht werden.

Dabei kann ein einzelner Marker verwendet werden, oder aber eine Mehrzahl von gleichen Markern, welche nicht voneinander unterscheidbar sind (also von Markern derselben Sorte, das heisst Marker welche dieselbe charakteristische Eigenschaft mit demselben Wert aufweisen). Ist in der Anmeldung von einem Marker die Rede, ist eine Mehrzahl von Markern derselben Sorte implizit mitgemeint sofern dies nicht unsinnig sein sollte.

Eine Mehrzahl von Markern derselben Sorte kann die Rückverfolgbarkeit des Objekts erleichtern und/oder verbessern, indem beispielsweise ein Marker von mehreren Markern auf einem Objekt schneller gefunden werden kann als ein einzelner Marker auf dem Objekt. Zum Beispiel kann eine Mehrzahl von Markern auf einem Objekt ein stärkeres Messsignal eines Messverfahrens der charakteristischen Eigenschaft des Markers liefern kann als ein einzelner Marker auf dem Objekt. Durch eine Mehrzahl von Markern derselben Sorte kann die Rückverfolgbarkeit verbessert werden, weil ein Entfernen oder Beschädigen eines einzelnen am Objekt befestigten Markers durch das Vorhandensein anderer Marker derselben Sorte an dem Objekt kompensiert werden kann.

Eine Einlagerungsstelle bezeichnet eine Stelle am Schmuckstein, welche in Innern des Schmucksteins angeordnet ist und welche genug Platz für einen Marker aufweist. Mit anderen Worten ist die Einlagerungsstelle ein räumliches Volumen an einer Position im Schmuckstein, welche nicht an der Aussenoberfläche des Schmucksteins liegt. Die Einlagerungsstelle weist mindestens ein räumliches Volumen der Grösse eines Markers auf. Die Einlagerungsstelle ist beispielsweise ein Riss, ein Mikroriss, eine Fissur, ein Spalt, ein Kratzer, eine Vertiefung, eine Fehlstelle, ein Defekt, eine Blase, eine Kavität, ein Kristallgitterdefekt und/oder eine Wegbarkeit bedingt durch die Kristallstruktur oder das Kristallwachstum, wie z.B. Zwillingsflächen oder Subkorngrenzen.

Insbesondere ist die Einlagerungsstelle ein Riss.

Ein Riss in einem Schmuckstein ist eine schmale Öffnung im Schmuckstein, welche sich von der Aussenoberfläche des Schmucksteins ins Innere des Schmucksteins erstreckt. Dabei ist eine Tiefe des Risses mindestens um einen Faktor fünf grösser als eine Breite des Risses, insbesondere um einen Faktor 10 grösser. Insbesondere ist der Riss um einen Faktor 50 tiefer als breit. Eine Länge des Risses kann variieren, ist aber immer grösser als die Breite des Risses. Länge und Breite des Risses verlaufen beispielsweise entlang der Oberfläche des Schmucksteins. Auch ein Mikroriss ist ein Riss.

Insbesondere ist die Einlagerungsstelle im Schmuckstein natürlich ausgebildet.

Das heisst, dass beispielsweise natürlich entstandene Defekte des Schmucksteins als Einlagerungsstelle verwendet werden. Auf diese Weise kann ein Schmuckstein unter Verwendung seiner natürlichen Eigenschaften rückverfolgbar gemacht werden, ohne dass der Schmuckstein durch das Rückverfolgbarmachen Schaden nimmt, in seiner Grundsubstanz manipuliert wird, strukturell verändert wird, optisch verändert wird und/oder sein finanzieller Wert reduziert wird.

Zusätzlich oder alternativ kann eine Einlagerungsstelle auch künstlich am Schmuckstein geschaffen werden. Beispielsweise kann eine Einlagerungsstelle bei einem Abbau eines natürlichen Schmucksteins entstehen, etwa bei einer Sprengung und/oder anderen Einflüssen wie Druckänderung, mechanische Be- und Entlastung und/oder Reibung während des Abbaus und/oder des Transports.

Mit Befestigen in der Einlagerungsstelle ist gemeint, dass der Marker positionsfest relativ zum Schmuckstein in der Einlagerungsstelle angeordnet wird. Der Marker wird also in der Einlagerungsstelle unbeweglich fixiert.

Der Marker ist insbesondere lösbar in der Einlagerungsstelle befestigt.

Nach dem Befestigen kann der Marker also beispielsweise wieder aus der Einlagerungsstelle gelöst werden. Je nach Art der Befestigung kann das Lösen des Markers aus der Einlagerungsstelle von einer bestimmten Technik und/oder bestimmten Umständen bzw. Randbedingungen abhängen. Beispielsweise kann ein spezifischer Befestigungsstoff nur mit einem spezifischen Lösungsmittel gelöst werden.

Insbesondere ist der Marker unlösbar in der Einlagerungsstelle befestigt.

Mit Erfassen einer Markierungsinformation ist gemeint, dass mindestens eine bestimmte Information, nämlich welcher eindeutig identifizierbare Marker in der Einlagerungsstelle welchen Schmucksteins befestigt wurde, wieder abrufbar zur Verfügung gestellt wird. Die Markierungsinformation kann auch weitere Informationen umfassen, beispielsweise Zeitpunkt, Ort bzw. Koordinaten und/oder weitere Informationen (wie Namen von natürlichen oder juristischen Personen, Bemerkungen, Zahlenwerte) betreffend die Gewinnung, Markierung, Verarbeitung, Transport, Verkauf des Schmucksteins und/oder auch ganz anderer Natur.

Durch das erfindungsgemässe Verfahren wird es möglich, einen Schmuckstein rückverfolgbar zu machen. Der Marker ist für das blosse menschliche Auge nicht sichtbar, und dadurch wird der Schmuckstein optisch nicht beeinträchtigt durch das Rückverfolgbarmachen. Der unsichtbare Marker kann auf diese Weise unbegrenzt lange am Schmuckstein befestigt bleiben, ohne dessen Erscheinungsbild zu verändern oder sogar zu beeinträchtigen. Eine Veränderung oder sogar Beeinträchtigung des Erscheinungsbilds des Schmucksteins könnte dessen Wert verringern. Umgekehrt kann der Wert eines rückverfolgbaren Schmucksteins im Vergleich zu einem nicht rückverfolgbaren Schmuckstein zunehmen, vor allem auch wenn das Rückverfolgbarmachen sein Erscheinungsbild nicht beeinträchtigt oder verändert.

Der Marker wird in einer Einlagerungsstelle des Schmucksteins befestigt, wodurch er im Inneren des Schmucksteins angeordnet ist. Durch diese Positionierung in der Einlagerungsstelle ist der Marker geschützt vor Verarbeitungsschritten und/oder Manipulationsversuchen.

Durch die Unsichtbarkeit des Markers von blossen Auge und dessen dadurch ermöglichten nicht störenden und beliebig langen Verbleibs in der Einlagerungsstelle des Schmucksteins kann der Schmuckstein ab dem Zeitpunkt des Befestigens des Markers immer eindeutig identifiziert werden. Ein zwischenzeitliches Entfernen des Identifikationsmerkmals - also letztendlich der Markers - für eine Bearbeitung und oder eine finale Verwendung (beispielsweise in einer Fassung als Schmuckstück) wie im Stand der Technik fällt weg. Das zwischenzeitliche Entfernen kann dabei bewusst vorgenommen werden wie bei der Hülle im oben zitierten Stand der Technik. Oder das zwischenzeitliche Entfernen geschieht ohnehin, beispielsweise beim Schleifen eines Schmucksteins und dem daraus resultierenden Entfernen einer Gravur oder Ätzung der Oberfläche des Schmucksteins. Der Verbleib des Markers in der Einlagerungsstelle und dadurch im Schmuckstein macht das Verfahren zuverlässig, sicher und manipulationsresistent.

Analog gilt auch für die Positionierung des Markers in der Einlagerungsstelle und den daraus resultierenden Schutz vor Verarbeitungsschritten und/oder Manipulationen, dass dieses Verfahren zum Rückverfolgbarmachen zuverlässig, sicher und/oder manipulationsresistent ist.

Und gerade im Zusammenspiel dieser beiden Merkmale (nämlich unsichtbarer Marker und Befestigen des Markers in der Einlagerungsstelle) wird das beschriebene Verfahren zum Rückverfolgbarmachen besonders sicher. Der dauernde Verbleib des Markers im Schmuckstein - ermöglicht durch den unsichtbaren Marker, welcher ohne optisch nachteilig zu wirken unbegrenzt lange am Schmuckstein befestigt sein kann - und der Schutz des Markers vor Manipulation und/oder Verarbeitungsschritten - ermöglicht durch das Befestigen des Markers in der Einlagerungsstelle - machen das Verfahren zum Rückverfolgbarmachen besonders zuverlässig, sicher und/oder manipulationsresistent. Ohne die Unsichtbarkeit des Markers sollte der Marker nicht beliebig lange am Schmuckstein verbleiben und sollte somit auch nicht in die geschützte Einlagerungsstelle eingebracht werden. Und ohne den mechanischen Schutz der Einlagerungsstelle wäre der Marker weniger sicher und manipulationsresistent am Schmuckstein befestigt.

Beispielsweise kann das beschriebene Verfahren zum Rückverfolgbarmachen eines Schmucksteins auf einen Schmuckstein in verschiedenen Stadien seiner Verarbeitung angewendet werden. Das Verfahren kann auf einen unverarbeiteten Schmuckstein ebenso angewendet werden wie auf einen teilweise verarbeiteten Schmuckstein und/oder einen fertig verarbeiteten Schmuckstein. Dies ist von Vorteil, da ein Schmuckstein unabhängig von seinem Verarbeitungsgrad rückverfolgbar gemacht werden kann.

Auch eine mehrfache Anwendung des Verfahrens mit voneinander verschiedenen und dadurch unterscheidbaren Markern ist möglich. Beispielsweise können auf diese Weise verschiedene Handelswege und/oder Verarbeitungsstufen rückverfolgbar gemacht werden.

Insbesondere ist der Schmuckstein vor dem Befestigen des Markers in der Einlagerungsstelle des Schmucksteins von einem Schmuckstück umfasst.

Mit anderen Worten kann der Schmuckstein rückverfolgbar gemacht werden durch das beschriebene Verfahren, selbst wenn der Schmuckstein bereits schon ein einem Schmuckstück angeordnet ist. Dabei kann das Schmuckstück nur teilweise oder aber bereits schon fertig ausgebildet und/oder verarbeitet sein. Das beschriebene Verfahren kann also auch auf Schmucksteine in teilfertigen oder fertigen Schmuckstücken angewendet werden.

Durch das Rückverfolgbarmachen des Schmucksteins kann das diesen Schmuckstein umfassende Schmuckstück ebenso rückverfolgbar gemacht werden, sofern das Schmuckstück den Schmuckstein beim Rückverfolgbarmachen umfasst. Die Markierungsinformation kann auch Information zum Schmuckstück umfassen. Auf diese Weise kann ein Schmuckstück oder auch nur Teile eines Schmuckstücks rückverfolgbar gemacht werden. Auch auf eine Herkunft eines Schmucksteins aus einem Schmuckstück kann geschlossen werden. Gestohlene Schmucksteine und/oder Schmuckstücke können auf diese Weise identifiziert werden. Ein Schmuckstück kann die das Rückverfolgbarmachen etwa auch fälschungssicher gemacht werden.

Weitere Ausführungsformen gehen aus den abhängigen Patentansprüchen hervor.

Der Marker wird in einer Trägerflüssigkeit suspendiert zur Einlagerungsstelle transportiert, um den Marker in der Einlagerungsstelle des Schmucksteins zu befestigen.

Ein Suspendieren des Markers in einer Trägerflüssigkeit erlaubt einen einfachen und effizienten Transport zur Einlagerungsstelle. Suspendiert heisst, dass der Marker von der Trägerflüssigkeit im Wesentlichen umschlossen ist. Im Falle von mehreren Markern sind suspendierte Marker in der Trägerflüssigkeit verteilt. Der Marker kann in einer Trägerflüssigkeit suspendiert gut in die Einlagerungsstelle eingebracht werden. Die Trägerflüssigkeit kann auch helfen, mehrere Marker in einer gleichmässigen Verteilung in eine oder mehrere Einlagerungsstellen einzubringen.

Insbesondere ist die Trägerflüssigkeit bei Raumtemperatur flüssig.

Auf diese Weise kann der Marker bei Raumtemperatur einfach in der Trägerflüssigkeit suspendiert werden. Auch der Transport des Markers zur Einlagerungsstelle kann bei Raumtemperatur erfolgen, was einen geringen technischen Aufwand bedeutet.

Insbesondere ist die Trägerflüssigkeit bei Raumtemperatur in einem festen Aggregatszustand. Beispielsweise liegt die Trägerflüssigkeit bei Raumtemperatur in Pulverform vor.

Die Trägerflüssigkeit ist beispielsweise erst bei einer Temperatur von mindestens 80 Grad Celsius flüssig. Insbesondere ist die Trägerflüssigkeit erst bei einer Temperatur von mindestens 150 Grad Celsius flüssig. Insbesondere ist die Trägerflüssigkeit erst bei einer Temperatur von mindestens 300 Grad Celsius flüssig.

Ein Beispiel für eine Trägerflüssigkeit mit festem Aggregatszustand bei Raumtemperatur ist Glas, und insbesondere Bleiglas. Bei Bleiglas handelt es sich um Glas mit einer relativ geringen Schmelztemperatur und mit verschiedener Zusammensetzungen, welchem Blei hinzugefügt ist. Das Blei im Glas erhöht die Lichtbrechung und gleicht diese derjenigen eines Schmucksteins an, beispielsweise derjenigen eines Rubins.

Optional umfasst die Trägerflüssigkeit ein Flussmittel für einen Schmuckstein. Insbesondere ist die Trägerflüssigkeit ein Flussmittel für einen Schmuckstein.

Ein Flussmittel für einen Schmuckstein erlaubt, den Schmuckstein bei einer relativ tiefen Temperatur wenigstens teilweise anzulösen bzw. aufzulösen. Anders ausgedrückt wird durch das Flussmittel die Schmelztemperatur des Schmucksteins verringert. Eine andere Bezeichnung für Flussmittel ist Fluxmittel. Bei einer Anwendung von Flussmittel wird also der Schmelzpunkt eines Schmucksteins, beispielsweise eines Korundes (wie etwa eines Rubins und Saphirs) herabgesetzt, sodass dieser Schmuckstein bei niedrigeren Temperaturen als ohne Flussmittel schmilzt und insbesondere bei niedrigeren Temperaturen als ohne Flussmittel partiell schmilzt.

Flussmittel können beim sogenannten Brennen oder Erhitzen eines Schmucksteins eingesetzt werden, um Makel des Schmucksteins einfacher und rascher zu verringern und/oder zu beseitigen, als dies beim Brennen bzw. Erhitzen ohne Flussmittel erfolgen würde. Makel des Schmucksteins können potentielle Einlagerungsstellen sein, also beispielsweise ein Riss, ein Mikroriss, eine Fissur, ein Spalt, ein Kratzer, eine Vertiefung, eine Fehlstelle, ein Defekt, eine Blase, eine Kavität, ein Kristallgitterdefekt und/oder eine Wegbarkeit bedingt durch die Kristallstruktur oder das Kristallwachstum, wie z.B. Zwillingsflächen oder Subkorngrenzen. Durch das Flussmittel kann eine Teilschmelze des Schmucksteins (also eine durch ein partielles Schmelzen des Schmucksteins hervorgebrachte Schmelze) besser zu Stellen mit den Makeln gelangen und Defekte besser verringern oder beheben, als dies ohne Flussmittel der Fall ist.

Das Flussmittel ist beim Brennen flüssig und kann mindestens einen Teil der Trägerflüssigkeit ausmachen, in welcher der Marker suspendiert ist, um zur Einlagerungsstellte transportiert zu werden.

Optional umfasst die Trägerflüssigkeit eine Schmelze aufweisend ein Flussmittel für einen Schmuckstein sowie eine Teilschmelze des Schmucksteins. Insbesondere ist die Trägerflüssigkeit eine Schmelze aufweisend ein Flussmittel für einen Schmuckstein sowie eine Teilschmelze des Schmucksteins.

Eine Schmelze, welche sowohl geschmolzenen Schmuckstein (insbesondere von einem partiellen Schmelzen des Schmucksteins herrührend) als auch Flussmittel umfasst, kann Makel des Schmucksteins einfach und rasch verringern und/oder beseitigen.

Optional verbleibt das Flussmittel nach einem Erstarren einer partiellen Schmelzung des Schmucksteins (also nach erneutem Verfestigen einer Teilschmelze) mindestens teilweise und insbesondere vollständig im Schmuckstein.

Beispielsweise enthält oder ist ein Flussmittel für Rubine Borax.

Vorteilhafterweise kann ein Flussmittel, welches in einem Verarbeitungsschritt des Schmucksteins sowieso eingesetzt wird, mindestens teilweise oder vollständig als Trägerflüssigkeit verwendet werden. Auf diese Weise kann das Verfahren zum Rückverfolgbarmachen des Schmucksteins mindestens teilweise mit Schritten einer Verarbeitung des Schmucksteins, insbesondere des Brennens, kombiniert werden. Dies spart Kosten, Zeit, Energie und Material.

Optional verbleibt die Trägerflüssigkeit nach dem Transport des Markers zur Einlagerungsstelle mindestens teilweise oder vollständig im Schmuckstein.

Optional wird die Trägerflüssigkeit nach dem Transport des Markers zur Einlagerungsstelle mindestens teilweise oder vollständig aus dem Schmuckstein entfernt.

Alternativ kann der Marker auch frei von einer Trägerflüssigkeit zur Einlagerungsstelle transportiert werden. Beispielsweise kann der Marker in Pulverform eingeblasen werden. Ein Transport des Markers durch elektromagnetische Felder und/oder mittels ionisierender Strahlung ist ebenso denkbar - was sowohl mit als auch ohne Trägerflüssigkeit machbar ist.

Optional wird der Marker nach dem Transport zur Einlagerungsstelle in der Einlagerungsstelle befestigt, indem der Schmuckstein partiell geschmolzen wird und sich wieder verfestigt. Insbesondere wird dabei ein Flussmittel verwendet.

Optional wird der bereits in der Einlagerungsstelle befestigte Marker zusätzlich im Schmuckstein befestigt, indem der Schmuckstein partiell geschmolzen wird und sich wieder verfestigt. Insbesondere wird dabei ein Flussmittel verwendet.

Optional wird der Marker nach dem Transport zur Einlagerungsstelle in der Einlagerungsstelle befestigt, indem sich eine Schmelze umfassend schmucksteinfremdes Material in einem Bereich um den Marker verfestigt. Insbesondere umfasst oder ist das schmucksteinfremde Material Glas, beispielsweise Bleiglas.

Optional wird der bereits in der Einlagerungsstelle befestigte Marker zusätzlich im Schmuckstein befestigt, indem sich eine Schmelze umfassend schmucksteinfremdes Material in einem Bereich um den Marker verfestigt. Insbesondere umfasst oder ist das schmucksteinfremde Material Glas, beispielsweise Bleiglas.

Der Schmuckstein wird mindestens teilweise in die Trägerflüssigkeit mit dem suspendierten Marker eingetaucht, um den in der Trägerflüssigkeit suspendierten Marker zur Einlagerungsstelle im Schmuckstein zu transportieren. Insbesondere kann der Schmuckstein vollständig in der Trägerflüssigkeit eingetaucht sein.

Ein mindestens teilweises Eintauchen des Schmucksteins in die Trägerflüssigkeit erlaubt einen einfachen, kostengünstigen, effizienten, energiesparsamen und raschen Transport des Markers zur Einlagerungsstelle. Bei mehreren Markern kann durch das Eintauchen eine gleichmässige Verteilung der Marker erreicht werden.

Der Schmuckstein wird über eine Zeitdauer von mehreren Stunden mindestens teilweise in der Trägerflüssigkeit eingetaucht. Zusätzlich wird der Schmuckstein und die Trägerflüssigkeit in Bewegung versetzt, also beispielsweise geschüttelt, beschallt (insbesondere mit Ultraschall), geschwenkt, rotiert oder gerührt.

In einer nicht beanspruchten Alternative kann der Schmuckstein auch mit der Trägerflüssigkeit besprüht werden. In einer anderen nicht beanspruchten Alternative kann die Trägerflüssigkeit auch in die Einlagerungsstellen injiziert also eingespritzt werden.

Insbesondere kann ein Transport des Markers zur Einlagerungsstelle unter von einem Umgebungsdruck unterschiedlichen Transportdruck erfolgen. Mit Umgebungsdruck ist ein Luftdruck einer Umgebung gemeint.

Beispielsweise kann der Transport des Markers zur Einlagerungsstelle unter Überdruck erfolgen, also einem Druck höher als dem Umgebungsdruck. Der Transportdruck ist in diesem Fall ein Überdruck. Der Überdruck kann dabei um mindestens einen Faktor 1.5 grösser sein als der Umgebungsdruck. Insbesondere ist der Überdruck um mindestens einen Faktor 5 grösser als der Umgebungsdruck.

Insbesondere ist der Überdruck um mindestens einen Faktor 10 grösser als der Umgebungsdruck.

Beispielsweise kann der Transport des Markers zur Einlagerungsstelle unter Unterdruck erfolgen, also einem Druck tiefer als dem Umgebungsdruck. Der Transportdruck ist in diesem Fall ein Unterdruck. Der Unterdruck kann dabei um mindestens einen Faktor 1.5 kleiner sein als der Umgebungsdruck. Insbesondere ist der Unterdruck um mindestens einen Faktor 5 kleiner als der Umgebungsdruck. Insbesondere ist der Unterdruck um mindestens einen Faktor 10 kleiner als der Umgebungsdruck.

Beispielsweise entspricht der Transportdruck einem Druck in einem von einer Umgebung abgetrennten Transportdruckraum, in welchem sich mindestens ein Teil des Schmucksteins und der Marker befinden, während der Marker zur Einlagerungsstelle transportiert wird.

Insbesondere befindet sich der ganze Schmuckstein und der Marker in einem von einer Umgebung abgetrennten Transportdruckraum, in welchem Transportdruck herrscht, während der Marker zur Einlagerungsstelle transportiert wird.

Beispielsweise herrscht der Transportdruck also in einer Druckkammer, welcher dann auch als Transportdruckraum bezeichnet werden kann.

Beispielsweise herrscht der Transportdruck lediglich in einem Transportdruckbereich, welcher den Marker und die Einlagerungsstelle umfasst, wobei der Transportdruckbereich als Teil der Umgebung ausgebildet ist.

Anders gesagt ist der Transportdruckbereich frei von einer Trennung von einer Umgebung ausgebildet. In diesem Transportdruckbereich befindet sich mindestens ein Teil des Schmucksteins und der Marker, während der Marker zur Einlagerungsstelle transportiert wird. Beispielsweise kann durch gezieltes Ausrichten eines Fluidstrahls auf und/oder um den Schmuckstein ein Transportdruckbereich in einer Umgebung um die Einlagerungsstelle zur Verfügung gestellt werden, in welchem der Marker zur Einlagerungsstelle transportiert wird.

Optional wird der Marker in einer Isopropanol umfassenden Trägerflüssigkeit suspendiert. Insbesondere wir der Marker in einer mindestens 50% Gewichtsanteil Isopropanol umfassenden Trägerflüssigkeit suspendiert.

Isopropanol ist kostengünstig. Isopropanol weist für den Transport der Marker in räumlich begrenzte und schwer erreichbare Einlagerungsstellen gute Transporteigenschaften wie Kriechverhalten und Oberflächenbenetzung auf. Isopropanol ist nicht giftig und einfach in der Anwendung. Isopropanol verdunstet und verdampft rasch und rückstandsfrei.

Alternativ kann auch eine Trägerflüssigkeit frei von Isopropanol verwendet werden. Beispielsweise kann eine ethanolbasierte Trägerflüssigkeit verwendet werden.

Insbesondere wird die Trägerflüssigkeit verdunstet oder verdampft, nachdem der Marker in die Einlagerungsstelle transportiert geworden ist.

Optional wird vor dem Befestigen des Markers in der Einlagerungsstelle des Schmucksteins die Trägerflüssigkeit mit einem Zusatzstoff versetzt, wobei der Zusatzstoff insbesondere ein Primer ist.

Ein Zusatzstoff in der Trägerflüssigkeit kann beim Befestigen des Markers eine unterstützende oder sogar tragende Rolle spielen. Ein Primer ist ein Stoff, welcher sowohl dem Schmuckstein als auch dem Marker gut anhaftet. Ein Primer wird auch als Haftvermittler bezeichnet. Anders ausgedrückt erstellt ein Primer in der Grenzfläche zwischen Marker und Schmuckstein eine enge physikalische oder chemische Bindung.

Alternativ kann die Trägerflüssigkeit auch frei von einem Zusatzstoff sein. Die Trägerflüssigkeit kann frei von einem Primer sein.

Optional umfasst der Zusatzstoff der Trägerflüssigkeit Silizium. Optional umfasst der Zusatzstoff der Trägerflüssigkeit Silan und/oder Ausgangsstoffe, aus welchen Silan entstehen kann.

Optional bildet sich vor und/oder bei einem Verdunsten oder Verdampfen der Trägerflüssigkeit eine Oberflächenbeschichtung mindestens einer Teiloberfläche der Einlagerungsstelle aus. Durch diese Oberflächenbeschichtung wird der Marker in der Einlagerungsstelle des Schmucksteins befestigt. Insbesondere umfasst die Oberflächenbeschichtung Silizium. Insbesondere umfasst die Oberflächenbeschichtung eine Silikatschicht.

Durch das Ausbilden einer Oberflächenbeschichtung (mindestens an einem Teil der Oberfläche) der Einlagerungsstelle kann der Marker stabil und sicher an der Einlagerungsstelle befestigt werden. Die Oberflächenbeschichtung kann durch den Zusatzstoff gebildet werden (beispielsweise durch Anhaften von Zusatzstoffpartikeln) und/oder aus einem Material, welches durch den Zusatzstoff entsteht (beispielsweise kann der Zusatzstoff eine Grundlage für ein Entstehen einer Oberflächenbeschichtung sein, wie etwa eine Flüssigkeit mit den entsprechenden Ausgangsstoffen). Beispielsweise kann die Trägerflüssigkeit selber beim Verdunsten oder Verdampfen eine Oberflächenbeschichtung ausbilden.

Optional wird der bereits in der Einlagerungsstelle befestigte Marker zusätzlich im Schmuckstein befestigt, indem eine Oberflächenbeschichtung mindestens an einem Teil der Oberfläche der Einlagerungsstelle ausgebildet wird.

Beispielsweise kann das Befestigen des Markers in der Einlagerungsstelle durch das Verfestigen einer Substanz aufgrund einer chemischen Reaktion einer oder mehrerer Komponenten erfolgen. Die chemische Reaktion zum Verfestigen der Substanz kann mit oder ohne Bestrahlung der Substanz erzeugt werden, insbesondere mit oder ohne Bestrahlung durch UV-Licht. Das Verfestigen der Substanz aufgrund der chemischen Reaktion kann eine Oberflächenbeschichtung mindestens eines Teils der Oberfläche der Einlagerungsstelle ausbilden. Alternativ kann das Verfestigen der Substanz aufgrund der chemischen Reaktion den Marker in der Einlagerungsstelle befestigen, wobei die Oberfläche der Einlagerungsstelle frei von einer teilweisen oder ganzen Oberflächenbeschichtung bleibt.

Optional wird der bereits in der Einlagerungsstelle befestigte Marker zusätzlich im Schmuckstein befestigt, indem das Verfestigen einer Substanz aufgrund einer chemischen Reaktion einer oder mehrerer Komponenten erfolgt.

Optional wird vor dem Befestigen des Markers in der Einlagerungsstelle des Schmucksteins eine Oberfläche des Markers behandelt.

Der Zeitpunkt der Behandlung der Oberfläche des Markers liegt dabei zu oder nach einem Beginn des Verfahrens zum Rückverfolgbarmachen, also nach einem Zeitpunkt einer Herstellung des Markers. Insbesondere gehört die Herstellung des Markers nicht zum Verfahren des Rückverfolgbarmachens. Insbesondere wird die Oberfläche des Markers vor dem Suspendieren in der Trägerflüssigkeit behandelt. Insbesondere wird die Oberfläche des Markers in der Trägerflüssigkeit behandelt.

Durch eine Behandlung der Oberfläche des Markers kann das Befestigen des Markers einfach, stabil und/oder dauerhaft erfolgen. Das Befestigen des Markers kann durch eine behandelte Oberfläche vereinfacht werden. Das Behandeln der Oberfläche kann beispielsweise bedeuten, dass die chemische und/oder physikalische Oberflächenbeschaffenheit verändert wird, und/oder beispielsweise der Marker eine elektrische Ladung erhält. Insbesondere eine negativ elektrische Ladung ist denkbar. Auch eine Magnetisierung des Markers kann vorgenommen werden. Die Oberfläche des Markers kann aber auch unbehandelt bleiben vor dem Befestigen in der Einlagerungsstelle.

Optional ist der Marker im Wesentlichen als Partikel ausgebildet.

Mit Partikel sind Teilchen gemeint, also kleine Festkörper. Beispielsweise sind Partikel feste Bestandteile von Aerosolen, Suspensionen oder Pulvern.

Im Wesentlichen als Partikel ausgebildet ist ein Marker, wenn mindestens 70% seines Volumens ein Partikel ist. Insbesondere ist ein Marker im Wesentlichen als Partikel ausgebildet, wenn mindestens 80% seines Volumens ein Partikel ist. Insbesondere ist ein Marker im Wesentlichen als Partikel ausgebildet, wenn mindestens 90% seines Volumens ein Partikel ist.

Optional ist der Marker im Wesentlichen kugelförmig ausgebildet. Insbesondere ist der Marker verkapselt ausgebildet.

Im Wesentlichen kugelförmig ausgebildet ist ein Marker, wenn in radialer Richtung des Markers vom Massenmittelpunkt des Markers aus der kleinste Radius maximal 10% kleiner ist als der grösste Radius. Insbesondere ist ein Marker im Wesentlichen kugelförmig ausgebildet, wenn der kleinste Radius maximal 20% kleiner ist als der grösste Radius. Insbesondere ist ein Marker im Wesentlichen kugelförmig ausgebildet, wenn der kleinste Radius maximal 30% kleiner ist als der grösste Radius.

Der Marker kann auch quaderförmig, zylinderförmig, n-eckig, tetraederförmig, kegelförmig oder ellipsoid ausgebildet sein. Der Marker kann auch eine unregelmässige Form aufweisen. Beispielsweise kann der Marker bereichsweise verschiedene oben genannte Formen kombinieren.

Verkapselt ausgebildet ist ein Marker dann, wenn der Marker als Behälter mit einem Inhalt ausgebildet ist.

Der Marker weist eine Grösse von 10nm bis und mit 1000nm auf. Insbesondere weist der Marker eine Grösse von 10nm bis und mit 500nm auf, und insbesondere von 20nm bis und mit 200nm auf.

Mit Grösse des Markers ist eine maximale Ausdehnung in derjenigen Dimension gemeint, in welcher der Marker am Grössten ist.

Optional weist der Marker eine elektrische Ladung auf. Insbesondere weist der Marker eine negative elektrische Ladung auf.

Eine elektrische Ladung des Markers (unabhängig davon, ob die Ladung von einer Oberflächenbehandlung des Markers herrührt oder auf andere Weise zustande kommt) kann bei der Befestigung des Markers in der Einlagerungsstelle helfen.

Alternativ ist der Marker elektrisch neutral.

Optional umfasst der Marker DNS, wobei der Marker durch die DNS eindeutig identifizierbar ist.

Optional ist die DNS künstlich zu dem Zwecke der Rückverfolgbarkeit hergestellt.

Die DNS ist in diesem Fall also ein Träger der charakteristischen Eigenschaft, anhand welcher der Marker eindeutig identifizierbar ist. Anders ausgedrückt ist die charakteristische Eigenschaft des Markers in Form von DNS ausgebildet, welche vom Marker umfasst wird. Die DNS kann anhand der darin gespeicherten Informationen identifiziert und von anderer DNS unterschieden werden.

Optional erfolgt das Erfassen der Markierungsinformation durch Speichern der Markierungsinformation in einem digitalen Logbuch.

Das Rückverfolgbarmachen des Schmucksteines kann durch eine digitale Form eines Logbuches erfolgen, unterstützt und/oder ergänzt werden. Unter digitalem Logbuch versteht sich eine digitale Technologie, welche es ermöglicht, die Markierungsinformation - umfassend beispielsweise auch die Weitergabe oder Bearbeitung eines Schmucksteines - zu erfassen, zu speichern und wiederzugeben. Zu jeder Markierungsinformation kann zusätzlich das Erfassungsdatum sowie gegebenenfalls auch die genaue Erfassungszeit erfasst werden. Diese digitale Art der Zeiterfassung kann auch Hashtag genannt werden. Der Hashtag lässt sich auch als ein digitaler Zeitstempel zu jedem Eintrag beschreiben. Durch wiederholtes Erfassen von Markierungsinformation, insbesondere in Kombination mit einem Hashtag, kann nicht nur die Markierungsinformation zum Zeitpunkt des Markierens rückverfolgbar gemacht werden, sondern auch die Markierungsinformation von nachfolgenden Schritten des Schmucksteines in seiner Handelskette. Dies können zum Beispiel Weiterverarbeitungsprozesse, Laborbegutachtungen, Goldschmiedearbeiten und Handelstätigkeiten sein. Das digitale Logbuch und damit die darin erfasste Markierungsinformation kann in einem Datennetzwerk wie beispielsweise im Internet auf zentralen und/oder dezentralen Datenspeichern gespeichert und/oder zugänglich gemacht werden.

Vor einem Eintrag in das digitale Logbuch kann der Schmuckstein identifiziert werden. Insbesondere kann dafür ein bereits zuvor am Schmuckstein befestigter Marker verwendet werden. Ist ein bereits am Schmuckstein befestigter Marker identifiziert (und dadurch auch der Schmuckstein), kann dieser Marker für die nächste Markierungsinformation dieses Schmucksteins weiterverwendet werden.

Alternativ oder zusätzlich können zum Identifizieren auch einzelne oder mehrere andere Merkmale des Schmucksteines genutzt werden wie beispielsweise optische, physikalische und/oder chemische Eigenschaften. Diese anderen Merkmale können zum Beispiel die Abmessungen, das Gewicht, mikroskopische und/oder makroskopische innere Merkmale und/oder die Farbe sein.

Ist das wiederholte Erfassen von Markierungsinformation zu demselben Schmuckstein (verifiziert durch eindeutiges Identifizieren des Schmucksteins vor dem Erfassen der nächsten Markierungsinformation) manipulationssicher gespeichert, kann mit einem einzigen Marker (bzw. einer einzigen Sorte von Markern) eine ganze Reihe von Schritten in dessen Handels- und Verarbeitungskette rückverfolgbar gemacht werden. Mit anderen Worten kann durch eindeutiges Identifizieren des Schmucksteins vor dem Erfassen der nächsten Markierungsinformation eine ganze Historie eines spezifischen Schmucksteins unter Verwendung eines einzigen Markers (oder einer einzigen Sorte von Markern) rückverfolgbar erfasst werden. Anders ausgedrückt ist nicht für jedes neue rückverfolgbare Ereignis bzw. jedes Erfassen von Markierungsinformation ein neuer Marker bzw. eine neue Sorte von Marker nötig (aber möglich). Insbesondere das wiederholte Erfassen von Markierungsinformation zu demselben Schmuckstein in einem digitalen Logbuch kann manipulationssicher ausgestaltet werden.

Der Zugang zu dem digitalen Logbuch kann durch verschiedene Technologien ermöglicht werden. Zum Beispiel sind dies Softwareprogramme und/oder Onlineapplikationen (z.B. Eingabe über eine Homepage oder Web-app) sein. Zum Beispiel können auch Programme für mobile Kommunikationsgeräte (Smartphones oder ähnliche Geräte) mit Internetzugang optimiert und zur Verfügung gestellt werden (apps).

Optional erfolgt das Erfassen der Markierungsinformation durch Verwendung von blockchain Technologie.

Das Erfassen der Markierungsinformation kann durch Anwendung einer blockchain Technologie erreicht werden. Die blockchain Technologie wird dabei verwendet, um das digitale Logbuch zu realisieren. Zum Beispiel wird dies durch eine dezentral aufgebaute digitale Technologie erreicht, welche auch als distributed (digital) ledger oder distributed blockchain Technology bezeichnet werden kann.

Die blockchain Technologie erlaubt eine manipulationssichere, günstige und vertrauenswürdige Bewirtschaftung eines digitalen Logbuchs.

Ein zweiter Aspekt dieser Erfindung ist ein Verfahren zum Rückverfolgen eines Schmucksteins, umfassend das Verfahren zum Rückverfolgbarmachen eines Schmucksteins wie oben beschrieben, sowie die darauf anschliessenden Schritte: Identifizieren des Markers,
Rückverfolgung des Schmucksteins durch Abgleichen des identifizierten Markers mit der erfassten Markierungsinformation.

Mit Hilfe des Verfahrens zum Rückverfolgbarmachen des Schmucksteins wie oben beschrieben wird der Schmuckstein vorbereitet, um die Rückverfolgung zu ermöglichen. Über das Identifizieren des Markers und das Abgleichen des identifizierten Markers mit der erfassten Markierungsinformation wird die Rückverfolgung durchgeführt. Die Vorteile und die Bedeutung des Rückverfolgens von Schmucksteinen sind weiter oben bereits beschrieben.

Im Folgenden wird der Erfindungsgegenstand anhand von bevorzugten Ausführungsbeispielen, welche in den beiliegenden Zeichnungen dargestellt sind, näher erläutert. Es zeigen jeweils schematisch:
- Figuren 1a-1c: Verfahren zum Rückverfolgbarmachen eines Schmucksteins;
- Figuren 2a-2f: Verfahren zum Rückverfolgbarmachen eines Schmucksteins unter Verwendung einer Trägerflüssigkeit;
- Figuren 3a-3f: Verfahren zum Rückverfolgbarmachen eines Schmucksteins unter Verwendung einer Trägerflüssigkeit und eines Zusatzstoffes.

Grundsätzlich sind in den Figuren gleiche Teile mit gleichen Bezugszeichen versehen.

Die Figuren la bis 1c zeigen ganz schematisch ein Verfahren zum Rückverfolgbarmachen eines Schmucksteins 2. Zuerst wird ein Marker 1 zur Verfügung gestellt, wie in Figur la dargestellt. Der Marker 1 ist in Figur la sehr stark vergrössert dargestellt; von blossem Auge ist der Marker 1 nicht sichtbar. Der Marker 1 ist ein im Wesentlichen kugelförmiges Partikel mit einem Durchmesser von 80nm, also ein Nanopartikel. Der Marker 1 umfasst DNS, welche zum eindeutigen Identifizieren des Markers 1 benutzt wird. Der Marker 1 umfasst neben der DNS Material auf der Basis von Silizium. Der Marker 1 ist in diesem Ausführungsbeispiel ein Typ von Markern, wie sie beispielsweise in der Patentanmeldung WO2013132014A1 beschrieben sind.

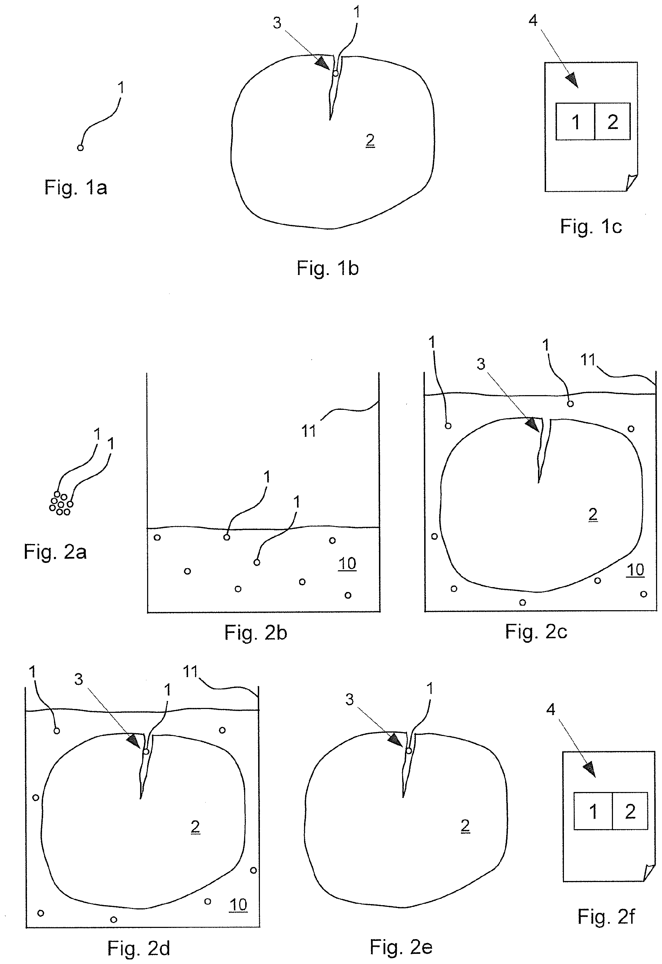

In Figur 1b ist der Schmuckstein 2 dargestellt, welcher im vorliegenden Beispiel ein Smaragd ist. Dieser Schmuckstein 2 weist eine Einlagerungsstelle 3 auf. In diesem Beispiel in Figur 1b ist die Einlagerungsstelle 3 grob vergrössert dargestellt und ist als Mikroriss oder auch Fissur im Schmuckstein 3 genannt ausgebildet. In dieser Einlagerungsstelle 3 des Schmucksteins 3 wird der Marker 1 befestigt. Wie in Figur 1c dargestellt, wird eine Markierungsinformation 4 erfasst, welche beinhaltet, dass der Marker 1 am Schmuckstein 2 befestigt worden ist. Die Markierungsinformation 4 wird typischerweise in einer elektronischen verfügbaren Datenbank abgelegt, welche über passende Sicherheitsmassnahmen gegen Manipulation, unbefugte Abfrage und/oder Datenverlust geschützt ist.

In den Figuren 2a bis 2f ist ganz schematisch ein Verfahren zum Rückverfolgbarmachen eines Schmucksteins 2 dargestellt, wobei eine Trägerflüssigkeit 10 verwendet wird, um den Marker 1 zur Einlagerungsstelle 3 zu transportieren. Marker 1, Schmuckstein 2 mit Einlagerungsstelle 3 sowie die Markierungsinformation 4 sind identisch mit denjenigen von Figur 1a bis 1c. Im in den Figuren 2a bis 2f dargestellten Verfahren werden mehrere Marker 1 (dargestellt in Figur 2a) verwendet. Dazu werden die Marker 1 in einer Trägerflüssigkeit 10 suspendiert, in diesem Fall in Isopropanol. Die Suspension aus Trägerflüssigkeit 10 und Markern 1 ist in Figur 2b in einem Gefäss 11 dargestellt.

Nach dem Suspendieren der Marker 1 in der Trägerflüssigkeit 10 wird der Schmuckstein 2 in dieser Suspension vollständig eingetaucht (siehe Figur 2c). Während der Schmuckstein 2 in der Suspension eingetaucht ist, wird das ganze Gefäss 11 bewegt, genauer gesagt mit einer Frequenz von 900 Hz bei Raumtemperatur in einem Shaker geschüttelt. Das Schütteln dauert 4 Stunden lang. Während dieser Zeit wird mindestens ein Marker 1 der Suspension dank der Trägerflüssigkeit 10 zur Einlagerungsstelle 3 transportiert, wie in Figur 2c dargestellt.

Nach dem Schütteln des Gefässes 11 wird der Schmuckstein 2 aus dem Gefäss 11 entfernt, und die Trägerflüssigkeit 10 kann bei Raumtemperatur verdunsten. Auf diese Weise wird der Marker 1 in der Einlagerungsstelle 3 des Schmucksteins 2 befestigt. Danach erfolgt analog zum Verfahren der Figuren 1a bis 1c das Erfassen der Markierungsinformation 4.

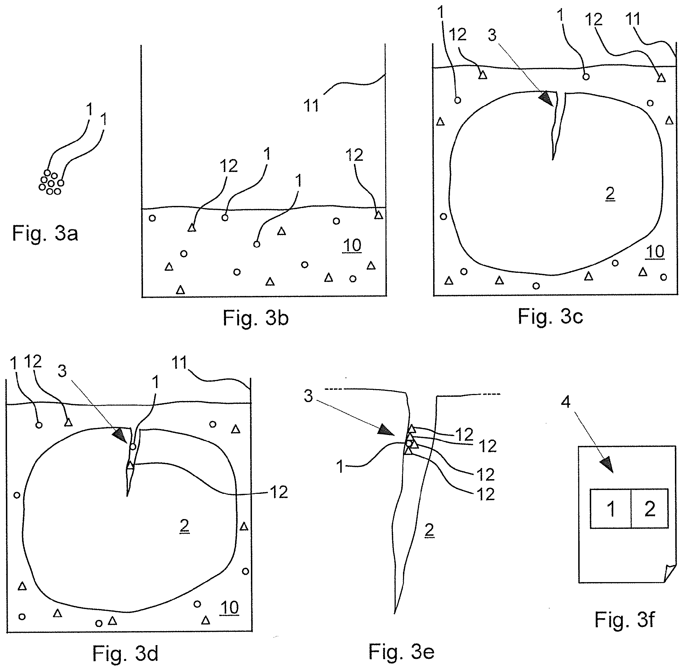

Die Figuren 3a bis 3f zeigen ein Verfahren analog zum Verfahren in den Figuren 2a bis 2f, mit dem Unterschied, dass die Suspension neben der Trägerflüssigkeit 10 und den Markern 1 auch noch den Zusatzstoff 12 umfasst. Der Zusatzstoff 12 ist in den Figuren 3b bis 3e als dreieckiges Symbol dargestellt. Der Zusatzstoff 12 ist im vorliegenden Fall aber kein Partikel, sondern besteht aus einer flüssigen Mischung aus Ammoniak, Tetraethylorthosilicat und purem Wasser. Dieser Zusatzstoff 12 in Form einer flüssigen Mischung dient als Grundlage für ein Ausbilden einer Silikatschicht. Entsprechend ist der Zusatzstoff 12 in der Suspension im Gefäss 11 in Figur 3b dargestellt, und ebenso in den Figuren 3c und 3d mit dem eingetauchten Schmuckstein 2. Auch der Zusatzstoff 12 wird durch die Trägerflüssigkeit 10 zur Einlagerungsstelle 3 transportiert, wie Figur 3d zeigt.

Figur 3e zeigt die Einlagerungsstelle 3 des Schmucksteins 2 schematisch und in sehr starker Vergrösserung. In Figur 3e ist der Schmuckstein 2 bereits aus dem Gefäss 11 entfernt, und die Trägerflüssigkeit 10 ist auch schon verdunstet. Aufgrund des Zusatzstoffes 12 hat sich auf einem Teil der Oberfläche der Einlagerungsstelle 3 eine Oberflächenbeschichtung ausgebildet, dargestellt durch eine Aneinanderreihung von dreieckigen Symbolen des Zusatzstoffes 12. Der Marker 1 ist durch die Oberflächenbeschichtung an der Einlagerungsstelle 3 des Schmucksteins 2 befestigt.

Die mit den unterschiedlichen oben beschriebenen Verfahren markierten Schmucksteine 2 sind in den Figuren 1b, 2e und 3e (in 3e allerdings nur als Ausschnitt) dargestellt. Diese Schmucksteine 2 weisen also einen Marker 2 auf. Zusammen mit der in den Figuren 1c, 2f und 3f symbolisch dargestellten Markierungsinformation 4 ist es möglich, diese Schmucksteine 2 rückzuverfolgen.

Das Verfahren zum Rückverfolgen eines Schmucksteins 2 umfasst die oben dargestellten Schritte des Verfahrens zum Rückverfolgbarmachen des Schmucksteins 2. Danach wird der rückverfolgbar gemachte Schmuckstein 2 untersucht, um den daran befestigten Marker 1 zu identifizieren. Dies erfolgt durch Lösen der DNS aus dem Marker 1, welcher sich in der Einlagerungsstelle 3 des Schmucksteins 2 befindet. Konkret wird ein ätzendes Lösungsmittel eingesetzt, um den Marker 1 wenigstens teilweise aufzulösen und dadurch die DNS freizugeben. Die DNS wird darauf durch qPCR (quantitative Echtzeit-PCR - ein Verfahren basierend auf eine Polymerase-Kettenreaktion zur Vervielfältigung der DNS sowie anschliessender Fluoreszenzmessung zur Quantifizierung der DNS) identifiziert. Auf diese Weise ist der Marker 1 identifiziert. Durch Heranziehen der Markierungsinformation 4 und entsprechendes Abgleichen kann durch die Identifizierung des Markers 1 und durch die von der Markierungsinformation 4 umfasste Information zum Marker 1 auf den korrespondierenden Schmuckstein 2 geschlossen werden. Der Schmuckstein 2 ist somit durch die in der Markierungsinformation 4 umfassten Informationen ebenso identifiziert, und das Verfahren zum Rückverfolgen abgeschlossen - denn der Schmuckstein 2 wurde zurückverfolgt bis zum Ereignis, dass der Schmuckstein 2 mit dem Marker 1 markiert geworden zu sein. Sofern die Markierungsinformation 4 entsprechend erfasst worden ist, kann auf Ort und Zeitpunkt der Anwendung des Verfahrens zum Rückverfolgbarmachen geschlossen werden, und/oder auf weitere Informationen wie etwa Herkunft aus einer Mine oder dergleichen (wie bereits weiter oben beschrieben).

## Patentansprüche

1. Verfahren zum Rückverfolgbarmachen eines Schmucksteins (2), umfassend die Schritte:
zur Verfügung stellen eines unsichtbaren Markers (1), welcher eine Grösse von 10 nm bis und mit 1000 nm aufweist, wobei der Marker (1) eindeutig identifizierbar ist,
Transport des in einer Trägerflüssigkeit (10) suspendierten Markers (1) zu einer Einlagerungsstelle (3), um den Marker (1) in der Einlagerungsstelle (3) des Schmucksteins (2) zu befestigen,
Befestigen des Markers (1) in der Einlagerungsstelle (3) des Schmucksteins (2), und
Erfassen einer Markierungsinformation (4), welche beinhaltet, dass dieser eindeutig identifizierbare Marker (1) an diesem Schmuckstein (2) befestigt ist, **dadurch gekennzeichnet, dass**
der Schmuckstein (2) mindestens teilweise in die Trägerflüssigkeit (10) mit dem suspendierten Marker (1) eingetaucht wird, um den in der Trägerflüssigkeit (10) suspendierten Marker (1) zur Einlagerungsstelle (3) im Schmuckstein (2) zu transportieren, wobei die Trägerflüssigkeit sich in einem Gefäss (11) befindet, und wobei der Schmuckstein (2) über eine Zeitdauer von mehreren Stunden mindestens teilweise in der Trägerflüssigkeit (10) eingetaucht ist und zusätzlich der Schmuckstein (2) und die Trägerflüssigkeit (10) in Bewegung versetzt werden, also beispielsweise geschüttelt, beschallt, insbesondere mit Ultraschall, geschwenkt, rotiert oder gerührt.

2. Verfahren gemäss Anspruch 1, **dadurch gekennzeichnet, dass** die Trägerflüssigkeit (10) ein Flussmittel für den Schmuckstein (2) umfasst.

3. Verfahren gemäss Anspruch 2, **dadurch gekennzeichnet, dass** der Marker (1) nach dem Transport zur Einlagerungsstelle (3) in der Einlagerungsstelle (3) befestigt wird, indem der Schmuckstein (2) partiell geschmolzen wird und sich wieder verfestigt.

4. Verfahren gemäss Anspruch 2 oder 3, **dadurch gekennzeichnet, dass** der Marker (1) nach dem Transport zur Einlagerungsstelle (3) in der Einlagerungsstelle (3) befestigt wird, indem sich eine Schmelze umfassend schmucksteinfremdes Material in einem Bereich um den Marker (1) verfestigt.

5. Verfahren gemäss einem der Ansprüche 1 bis 4, **dadurch gekennzeichnet, dass** vor dem Befestigen des Markers (1) in der Einlagerungsstelle (3) des Schmucksteins (2) die Trägerflüssigkeit (10) mit einem Zusatzstoff (12) versetzt wird, wobei der Zusatzstoff (12) insbesondere ein Primer ist.

6. Verfahren gemäss Anspruch 5, **dadurch gekennzeichnet, dass** sich vor und/oder bei einem Verdunsten oder Verdampfen der Trägerflüssigkeit (10) eine Oberflächenbeschichtung mindestens einer Teiloberfläche der Einlagerungsstelle (3) ausbildet, durch welche der Marker (1) in der Einlagerungsstelle (3) des Schmucksteins (2) befestigt wird, wobei die Oberflächenbeschichtung insbesondere Silizium umfasst.

7. Verfahren gemäss einem der Ansprüche 1 bis 6, **dadurch gekennzeichnet, dass** vor dem Befestigen des Markers (1) in der Einlagerungsstelle (3) des Schmucksteins (2) eine Oberfläche des Markers (1) behandelt wird.

8. Verfahren gemäss einem der Ansprüche 1 bis 7, **dadurch gekennzeichnet, dass** der Marker (1) im Wesentlichen als Partikel ausgebildet ist.

9. Verfahren gemäss einem der Ansprüche 1 bis 8, **dadurch gekennzeichnet, dass** der Marker (1) im Wesentlichen kugelförmig ausgebildet ist und insbesondere verkapselt ausgebildet ist.

10. Verfahren gemäss einem der Ansprüche 1 bis 9, **dadurch gekennzeichnet, dass** der Marker (1) eine elektrische Ladung aufweist, insbesondere eine negative elektrische Ladung.

11. Verfahren gemäss einem der Ansprüche 1 bis 10, **dadurch gekennzeichnet, dass** der Marker (1) DNS umfasst, wobei der Marker (1) durch die DNS eindeutig identifizierbar ist.

12. Verfahren zum Rückverfolgen eines Schmucksteins (2), umfassend das Verfahren zum Rückverfolgbarmachen eines Schmucksteins (2) gemäss einem der Ansprüche 1 bis 11, sowie die darauf anschliessenden Schritte:
Identifizieren des Markers (1),
Rückverfolgung des Schmucksteins (2) durch Abgleichen des identifizierten Markers (1) mit der erfassten Markierungsinformation (4).

## Claims

1. A method of rendering a gemstone (2) traceable, comprising the steps:
providing an invisible marker (1), wherein the marker (1) has a size of 10 nm up to and including 1000 nm, and wherein the marker (1) is uniquely identifiable,
transporting the marker (1) suspended in a carrier fluid (10) to a placement location (3) in order to fasten the marker (1) in the placement location (3) of the gemstone (2),
fastening the marker (1) in the placement location (3) of the gemstone (2), and
acquiring a marking information (4) which includes that this unambiguously identifiable marker (1) is attached to this gemstone (2),
**characterised in that**
the gemstone (2) is immersed at least partly into the carrier fluid (10) with the suspended marker (1), in order to transport the marker (1) which is suspended in the carrier fluid (10) to the placement location (3) in the gemstone (2), the carrier fluid being located in a vessel (11), and
wherein the gemstone (2) is at least partly immersed into the carrier fluid (10) over a time duration of several hours, and in addition the gemstone (2) and the carrier fluid (10) are brought into motion, thus for example shaken, exposed to sound, in particular with ultrasound, pivoted, rotated or stirred.

2. A method according to claim 1, **characterised in that** the carrier fluid (10) comprises a flow means for the gemstone (2).

3. A method according to claim 2, **characterised in that** the marker (1) is fastened in the placement location (3) after transport to the placement location (3) by way of the gemstone (2) being partially melted and resolidifying.

4. A method according to claim 2 or 3, **characterised in that** the marker (1) is fastened in the placement location (3) after transport to the placement location (3) by way of a melt which is foreign to the gemstone solidifying in a region around the marker (1).

5. A method according to one of claims 1 to 4, **characterised in that** an additive (12) is added to the carrier fluid (10) before the fastening of the marker (1) in the placement location (3) of the gemstone (2), wherein the additive (12) in particular is a primer.

6. A method according to claim 5, **characterised in that** a surface coating of at least a part-surface of the placement location (3) forms before and/or on evaporating or vapourising the carrier fluid (10), by way of which surface coating the marker (1) is fastened in the placement location (3) of the gemstone (2), wherein the surface coating in particular comprises silicon.

7. A method according to one of claims 1 to 6, **characterised in that** a surface of the marker (1) is treated before the fastening of the marker (1) in the placement location (3) of the gemstone (2).

8. A method according to one of claims 1 to 7, **characterised in that** the marker (1) is designed essentially as a particle.

9. A method according to one of the claims 1 to 8, **characterised in that** the marker (1) is designed in an essentially spherical manner and in particular in an encapsulated manner.

10. A method according to one of claims 1 to 9, **characterised in that** the marker (1) comprises an electrical charge, in particular a negative electrical charge.

11. A method according to one of claims 1 to 10, **characterised in that** the marker (1) comprises DNA, wherein the marker (1) is unambiguously identifiable by the DNA.

12. A method for tracing a gemstone (2), comprising the method for rendering a gemstone (2) traceable according to one of the claims 1 to 11, as well as the subsequent steps:
identifying the marker (1),
tracing the gemstone (2) by way of comparing the identified marker (1) with the acquired marking information (4).

## Revendications

1. Procédé de rendre une pierre précieuse (2) traçable, comprenant les étapes suivantes :
mise à disposition d'un marqueur (1) invisible, qui présente une taille de 10 nm à 1000 nm inclus, le marqueur (1) étant identifiable de manière univoque,
transport du marqueur (1) suspendu dans un liquide porteur (10) vers un site de dépôt (3), afin de fixer le marqueur (1) dans le site de dépôt (3) de la pierre précieuse (2),
fixation du marqueur (1) dans le site de dépôt (3) de la pierre précieuse (2), et
saisie d'une information de marquage (4) qui implique que ce marqueur (1) univoquement identifiable est fixé à cette pierre précieuse (2),
**caractérisé en ce que**
la pierre précieuse (2) est au moins partiellement immergée dans le liquide porteur (10) comprenant le marqueur (1) suspendu, afin de transporter le marqueur (1) suspendu dans le liquide porteur (10) jusqu'à le site de dépôt (3) dans la pierre précieuse (2), le liquide porteur (10) se trouvant dans un récipient (11), et
la pierre précieuse (2) étant au moins partiellement immergée dans le liquide porteur (10) pendant une durée de plusieurs heures et la pierre précieuse (2) et le liquide porteur (10) étant en outre mis en mouvement, c'est-à-dire par exemple secoués, sonorisés, en particulier par ultrasons, pivotés, tournés ou agités.

2. Procédé selon la revendication 1, **caractérisé en ce que** le liquide porteur (10) comprend un flux pour la pierre précieuse (2).

3. Procédé selon la revendication 2, **caractérisé en ce que** le marqueur (1) est fixé dans le site de dépôt (3) après le transport vers le site de dépôt (3) en faisant fondre partiellement la pierre précieuse (2) et en la solidifiant à nouveau.

4. Procédé selon la revendication 2 ou 3, **caractérisé en ce que** le marqueur (1) est fixé dans le site de dépôt (3) après le transport vers le site de dépôt (3) en solidifiant une masse fondue comprenant un matériau étranger à la pierre précieuse dans une zone autour du marqueur (1).

5. Procédé selon l'une des revendications 1 à 4, **caractérisé en ce qu'**avant la fixation du marqueur (1) dans le site de dépôt (3) de la pierre précieuse (2), le liquide porteur (10) est mélangé à un additif (12), l'additif (12) étant en particulier un primaire.

6. Procédé selon la revendication 5, **caractérisé en ce que**, avant et/ou lors d'une évaporation ou d'une vaporisation du liquide porteur (10), il se forme un revêtement de surface d'au moins une surface partielle du site de dépôt (3), par lequel le marqueur (1) est fixé dans le site de dépôt (3) de la pierre précieuse (2), le revêtement de surface comprenant en particulier du silicium.

7. Procédé selon l'une des revendications 1 à 6, **caractérisé en ce que**, avant de fixer le marqueur (1) dans le site de dépôt (3) de la pierre précieuse (2), une surface du marqueur (1) est traitée.

8. Procédé selon l'une des revendications 1 à 7, **caractérisé en ce que** le marqueur (1) se présente essentiellement sous forme de particules.

9. Procédé selon l'une des revendications 1 à 8, **caractérisé en ce que** le marqueur (1) est essentiellement sphérique et en particulier est encapsulé.

10. Procédé selon l'une des revendications 1 à 9, **caractérisé en ce que** le marqueur (1) présente une charge électrique, en particulier une charge électrique négative.

11. Procédé selon l'une des revendications 1 à 10, **caractérisé en ce que** le marqueur (1) comprend de l'ADN, le marqueur (1) étant univoquement identifiable par l'ADN.

12. Procédé de traçabilité d'une pierre précieuse (2), comprenant le procédé de rendre une pierre précieuse (2) traçable selon l'une des revendications 1 à 11, ainsi que les étapes suivantes:
identification du marqueur (1),
traçage de la pierre précieuse (2) par comparaison du marqueur identifié (1) avec les informations de marquage saisies (4).
